# EUROPEAN PATENT SPECIFICATION

(11) **EP 0 662 937 B1**
(45) Date of publication and mention of the grant of the patent: **19.03.1997**
(21) Application number: 93920061.4
(22) Date of filing: 13.08.1993
(51) Int. Cl.: C07C 1/26

(54) **PROCESS FOR CONVERTING 1,2-DICHLOROPROPANE TO PROPYLENE**
VERFAHREN ZUR UMWANDLUNG VON 1,2-DICHLORPROPAN IN PROPYLEN
PROCEDE DE CONVERSION DE 1,2-DICHLOROPROPANE EN PROPYLENE

(30) Priority: 01.10.1992 US 955173
(43) Date of publication of application: 19.07.1995
(73) Proprietor: THE DOW CHEMICAL COMPANY, Midland, Michigan 48640 (US)
(72) Inventor: ITO, Larry, N., Midland, MI 48640 (US); HARLEY, A., Dale, Baton Rouge, LA 70816 (US); HOLBROOK, Michael, T., Baton Rouge, LA 70808 (US); SMITH, David, D., Baton Rouge, LA 70809 (US); MURCHISON, Craig, B., Midland, MI 48640 (US); CISNEROS, Mark, D., Baton Rouge, LA 70810 (US)
(74) Representative: Burford, Anthony Frederick
(86) International application number: US9307658
(87) International publication number: WO9407819

(56) References cited:
- DD-A- 235 630
- US-A- 2 886 605

## Description

In the chlorohydrin process for making propylene oxide, 1,2-dichloropropane (PDC) is formed in significant quantities as a byproduct. Several attempts have been made in the art previously to convert this material to propylene, which can then be recycled to the chlorohydrin process, used in the manufacture of allyl chloride, or put to other beneficial uses or sold.

In DD-A-235,630 ("DE '630"), for example, PDC is converted to propylene in a catalytic gas phase reaction at temperatures ranging from 170 degrees Celsius to 450 degrees Celsius. The catalyst is described as an activated carbon which has been treated with a suspension of iron oxides and/or iron oxide hydrates, and then dried at temperatures in the range of 80 degrees to 200 degrees Celsius.

Other methods described in DE '630 include hydrogenation in the presence of a rhodium catalyst at 180-250 degrees Celsius, and the dechlorination of PDC at normal temperatures and in the presence of a pure titanium catalyst to a mixture (9:1) of propylene and chloropropylene.

The present invention is a novel and improved catalytic process for the conversion of 1,2-dichloropropane to propylene in a commercially substantial proportion (that is, at a yield (defined as the product of the conversion rate of 1,2-dichloropropane and the selectivity to propylene, on a hydrogen chloride- and hydrogen-free basis) of at least 10 percent, but preferably at least 15 percent and more preferably at least 35 percent), in which 1,2-dichloropropane (hereafter, PDC) is reacted with hydrogen in the presence of a supported catalyst including a selected Group IB metal or metals in an elemental or compound form with a selected Group VIII metal or metals, also in an elemental or compound form. Preferred catalysts will, however, consist of a combination of one or more Group IB metals in elemental or compound form with one or more Group VIII metals in elemental or compound form on a support.

With respect to the Group IB and Group VIII metals involved, preferred catalysts will comprise platinum in elemental or compound form as a Group VIII metal and copper in elemental or compound form as a Group IB metal. More preferably, the Group IB and Group VIII metals will consist of platinum and copper in their elemental or compound forms. Especially preferred is a supported bimetallic platinum/copper catalyst.

In terms of the amounts of platinum and copper employed in these preferred catalysts, platinum preferably comprises from 0.01 to 5.0 percent by weight of the catalyst (calculated on an elemental basis) and copper comprises from 0.01 to 15 percent by weight of the catalyst (also calculated on an elemental basis). The support can be any of those supports which are conventionally employed, but is preferably carbon or silica, with carbon being more preferred. High surface area carbons are still more preferred, for example a carbon having a specific surface area of at least 200 m²/g.

More preferably, the catalyst includes from 0.10 to 3.0 percent by weight of platinum and from 0.05 to 5 percent by weight of copper, and the carbon support has a specific surface area of at least 500 m²/g.

Most preferably, the catalyst includes from 0.20 to 1.0 percent by weight of platinum and from 0.1 to 2.0 percent by weight of copper, and the carbon support has a specific surface area of at least 800 m²/g. An example of a commercially-available carbon which has been found to be generally suited for use in the present invention is produced by Calgon Carbon Corporation under the designation "BPLF3", and may generally be characterized as having a specific surface area of 1100 m²/g to 1300 m²/g, a pore volume of 0.7 to 0.85 cm³/g, and an average pore radius of 12.3 to 14 angstroms (1.23 to 1.4 nm). Based on an X-ray fluorescence analysis of this carbon, a typical bulk composition of the BPLF3 carbon has been determined to be as follows (by weight percent): silicon, 1.5 percent; aluminum, 1.4 percent; sulfur, 0.75 percent; iron, 0.48 percent; calcium, 0.17 percent; potassium, 0.086 percent; titanium, 0.059 percent; magnesium, 0.051 percent; chlorine, 0.028 percent; phosphorus, 0.026 percent; vanadium, 0.010 percent; nickel, 0.0036 percent; copper, 0.0035 percent; chromium, 0.0028 percent; and manganese, 0.0018 percent (the remainder being carbon).

Preferably the catalyst for this process will have been pretreated by exposure to a chloride source, for example, hydrogen chloride, to improve initial selectivity to propylene over propane and to make the product stream immediately useful in an allyl chloride process (wherein impurity propane in the feed can react with chlorine to form 1-chloropropane, which because of a similarity in boiling points to allyl chloride is difficult to remove), for example, or to minimize venting in a propylene oxide process of any propane in the product stream fed or recycled to the propylene oxide process. In this regard, catalysts of the present invention which have been impregnated, dried and reduced under hydrogen have been observed to produce propane with decreasing selectivity and propylene with increasing selectivity over time. The initial chloride source pretreatment substantially reduces, however, the amounts of venting or downstream processing involved in making use of the product stream in a propylene oxide process or allyl chloride process, respectively.

Catalysts that have been pretreated by exposure to a chloride source and not reduced, or which have been merely dried and started up (and which have been in essence treated with hydrogen chloride in situ on start up), have been observed to produce the least amount of propane initially, albeit with a substantial penalty in conversion. For this reason, it is considered that it will generally be preferable to both reduce and pretreat (by exposure to a chloride source) a given catalyst, as opposed to reducing the catalyst solely, or not reducing the catalyst at all in favor of chloride-source pretreatment or treatment with HCl in situ on startup.

It will also be preferred to employ hydrogen chloride in the feed to reduce coking and catalyst deactivation rates. In this regard, the rate of conversion loss is preferably no more than 0.03 percent per hour, and especially no more than 0.01 percent per hour.

It will be appreciated that the conditions under which the process is carried out can vary, depending for example on whether the process is conducted in the liquid phase (for which both batchwise and continuous processes are contemplated) or in the gas phase as is presently preferred. It is expected that liquid phase processes can preferably be conducted at pressures ranging from atmospheric pressure up to 3000 psig (20.6 MPa (gauge)), temperatures of from 25 degrees Celsius up to 350 degrees Celsius, residence times of from one to 30 minutes and hydrogen to PDC molar feed ratios of from 0.1 to 1, to 100 to 1.

With respect to the preferred gas phase processes, the reaction pressure preferably ranges from atmospheric pressure up to 1500 psig (10.3 MPa (gauge)), more preferably ranges from 5 psig (0.03 MPa (gauge)) to 500 psig (3.4 MPa (gauge)), and most preferably ranges from 50 psig (0.34 MPa (gauge)) to 300 psig (2.1 MPa (gauge)).

The temperature will preferably be from 100 deg. C. to 350 deg. C., more preferably will be from 180 deg. C. to 300 deg. C., and most preferably will be from 200 deg. C. to 260 deg. C.

Preferred residence times will be from 0.25 seconds to 180 seconds, more preferably will be from 1.0 seconds to 20 seconds, and most preferably will be from 5 to 15 seconds.

Hydrogen to PDC feed ratios will preferably be (on a molar basis) from 0.1:1 to 100:1. More preferably, the hydrogen to PDC feed ratios will be from 0.3:1 to 10:1, and most preferably from 0.5:1 to 3:1. Preferably this process will on a commercial-scale further include a recycle stream comprised of any unreacted PDC and 2-chloropropane (as a by-product of the reaction), although as suggested above the amount of 2-chloropropane that is produced is preferably minimized by a chloride source pretreatment of the bimetallic Pt/Cu catalyst.

### Illustrative Examples

The present invention is more particularly illustrated by the examples which follow hereafter. In Examples 1-9, a number of catalyst preparations were evaluated for converting 1,2-dichloropropane to reaction products including propylene. Several parameters were measured and/or calculated for purposes of comparison, including PDC conversion (100 minus the mol percent of PDC in the test reactor effluent, excluding unreacted hydrogen and the hydrochloric acid produced by the reaction), selectivity to a given component (mols of component divided by mols PDC converted, multiplied by 100), liquid hourly space velocity (in hr⁻¹, the volume of liquid PDC fed per hour to the reactor divided by the packed bed volume of catalyst in the reactor), and catalyst productivity (on a basis of kilograms of propylene produced per hour per cubic meter of catalyst used).

For each example in Examples 1-9, PDC was converted to propylene by flowing hydrogen and PDC in the gas phase over a given catalyst to be evaluated. Liquid PDC was pumped via a piston pump through 1/16th inch (1.6 mm) (O.D.) nickel tubing to a Monel™ alloy (Huntington Alloys, Inco Alloys International, Inc.) gas sample cylinder packed with glass beads (unless specifically noted, all fittings and tubing were of Monel™ alloy). The 1/16th inch (1.6 mm) tubing extended to the center of the sample cylinder, with the sample cylinder being heated to a vaporization temperature of 110 degrees Celsius by electrical heat tracing. A thermocouple was used to monitor the skin temperature of the sample cylinder.

The flow of the hydrogen feed stream was controlled by a pre-calibrated mass flow controller. The desired flow of hydrogen was passed through the heated sample cylinder, where mixing of the gaseous PDC and hydrogen occurred. The mixed gases were then passed into a charged Monel™ tubular reactor (0.75 in. (1.9 cm.) O.D., 18 inches (45.7 cm.) in length) heated by ceramic lined electric elements to a desired reaction temperature.

The catalyst (1.2 cubic centimeters) was in each case charged into the reactor between 3 mm glass beads, and placed in the middle of the reactor. The catalyst was thereafter dried under a flow of nitrogen for one hour at 130 degrees Celsius, and then reduced under a 5:1 molar ratio of flowing nitrogen and hydrogen. In reducing the catalyst, the temperature was ramped up from 130 to 220 degrees Celsius at 3 degrees per minute, and then held at 220 degrees for a total reducing cycle time of about 2 hours.

Upon reaction of the mixed hydrogen and PDC in the reactor at a prescribed reaction temperature, the effluent from the reactor passed to a gas sampling valve, which provided gaseous aliquots for online gas chromatographic analysis in a Hewlett-Packard Model 5890 Series II gas chromatograph (Hewlett-Packard Company). The gas chromatograph was equipped with a flame ionization detector, and used 30 meter by 0.53 millimeter (I.D.) 100 percent methyl silicone/fused silica and 30 meter by 0.53 millimeter (I.D.) porous polymer-lined fused silica columns to separate the various reaction products. Response factors were conventionally determined by injections of gravimetrically-prepared standards of the individual reaction products. These response factors were applied in conjunction with individual peak areas and the total mols of all reaction products to determine the mol percents of individual components in the reactor effluent, and the selectivity to individual reaction products as described above.

### Example 1

For this example a bimetallic platinum-copper catalyst was prepared on a carbon support for comparison to platinum on a carbon support alone, to copper on a carbon support alone, and to the carbon support alone.

For the platinum-copper catalyst, an aqueous H₂PtCl₆ stock solution was prepared by dissolving 3.179 grams of H₂PtCl₆·6H₂O (J. T. Baker, Inc.; Baker Analyzed Grade, 37.6 percent Pt) in 100.00 mL of deionized and distilled water. 0.381 grams of CuCl₂ (Aldrich Chemical Company, Inc., 99.999 percent purity) were placed in a 250 mL Erlenmeyer flask, and 8.305 grams of the H₂PtCl₆ stock solution were added with swirling to dissolve the CuCl₂. The solution was then diluted with 42.64 grams of deionized, distilled water and swirled. 40.04 grams of Calgon BPLF3 activated carbon (6 x 16 mesh (3.4 mm x 1.2 mm), Calgon Carbon Corp., Pittsburgh, Pa.) were added to the flask, and the flask was agitated rapidly so that the carbon carrier was evenly coated with the aqueous Pt/Cu solution. The catalyst preparation was dried in an evaporating dish in air at ambient temperatures for 18 hours, and then further dried in an oven in air at 120 degrees Celsius for 2 hours before being charged to the reactor, dried and reduced. The resulting catalyst was comprised of 0.25 percent by weight of platinum (on an elemental basis) and 0.45 percent by weight of copper (also on an elemental basis).

Similar methods of preparation were used to make catalysts of 0.48 percent by weight of copper on the Calgon BPLF3 activated carbon support (omitting the chloroplatinic acid solution), and of 0.25 percent by weight of platinum on the same activated carbon (omitting the CuCl₂ from the process).

Reactions were run at various reaction temperatures on the apparatus described above with separate charges of each of these catalysts, as well as running reactions at various temperatures with an unimpregnated Calgon BPLF3 activated carbon support as a test for reactivity of the support.

Each of the catalysts was loaded into the 18 inch (45.7 cm) long tubular reactor described above, using about 1.2 cubic centimeters of catalyst. The liquid hourly space velocity (LHSV) was 1.25, and the hydrogen and PDC were fed to the reactor at a molar ratio of 6.0:1. Reactor temperatures, PDC conversions, productivities, and individual product selectivities for the runs with these catalysts are shown as follows in Table 1:

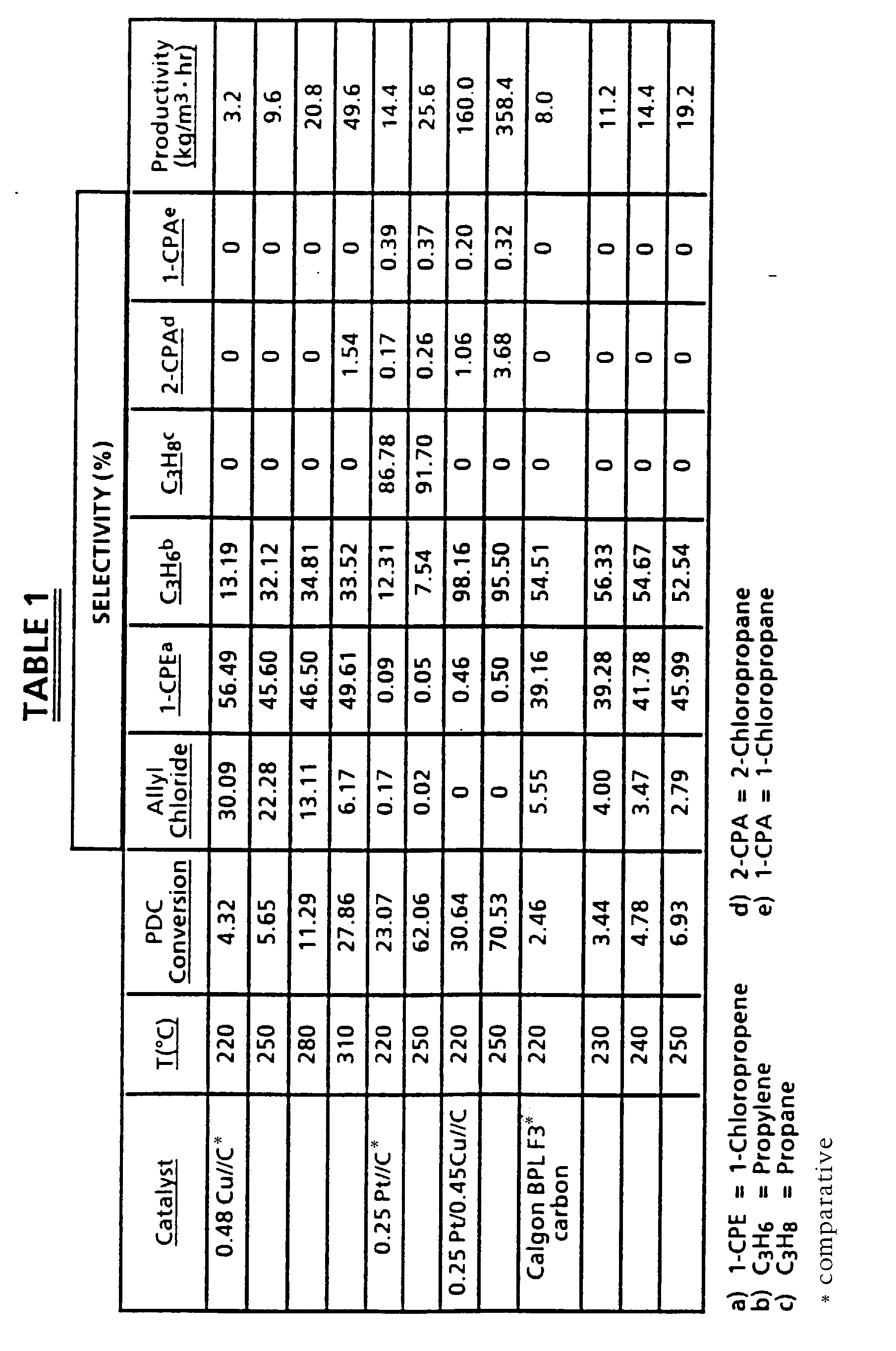

### Examples 2-6

In Examples 2-6, platinum-copper catalysts were prepared which used the same activated carbon support as in Example 1. The catalysts were each prepared, charged, dried and reduced as in Example 1, except that the catalysts for these examples used different amounts of platinum and copper (on an elemental basis).

In the runs with each of these catalysts, 1.2 cubic centimeter charges of a given catalyst were employed in the 18 inch (45.7 cm) Monel™ reactor, and the hydrogen to PDC feed ratio on a molar basis was set at 5:1 (rather than the 6:1 ratio seen in Example 1). Liquid hourly space velocities and reaction temperatures were varied. The results of these runs are shown in Table 2, and indicate propylene yields ranging under the conditions specified of from 28.5 percent (for the 0.5 Pt/12.3 Cu on carbon catalyst at 250 deg. C., or (36.7 x 77.7)/100) up to 64.2 percent (for the 0.28 Pt/3.42 Cu on carbon catalyst at 250 deg. C. and an LHSV of 0.30, or (87 x 73.8)/100):

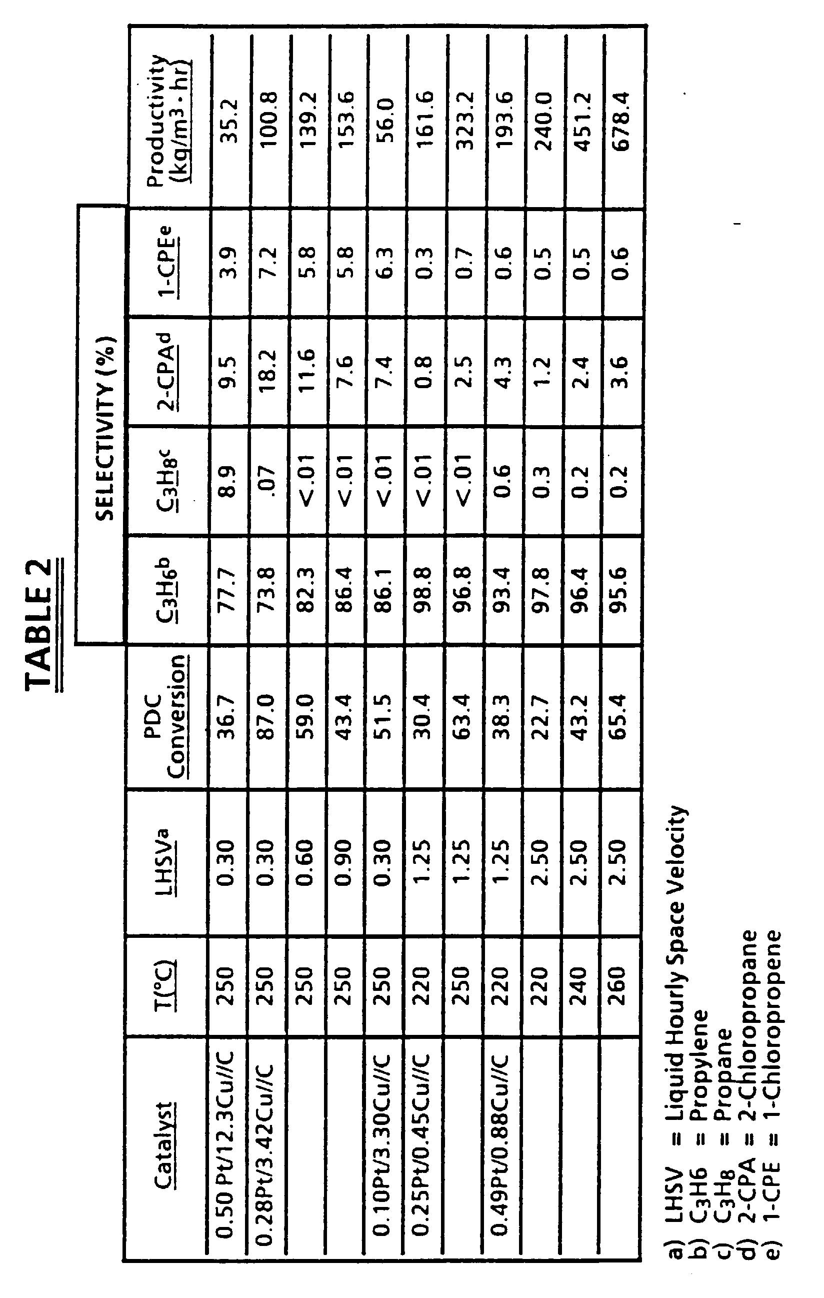

### Example 7

The catalyst prepared for this Example included palladium instead of platinum, along with the copper of previous examples.

In making the palladium-copper catalyst of this example, 0.393 grams of PdCl₂ (Aldrich Chemical Company, Inc., 99.999% purity) was dissolved in 2 mL of 12M hydrochloric acid (HCl) and diluted to 25.00 mL in a volumetric flask with distilled, deionized water. 0.097 grams of CuCl₂ (Aldrich Chemical Company, Inc., 99.999% purity) was placed in a 50 mL Erlenmeyer flask, and 1.435 grams of the prepared H₂PdCl₄ stock solution was added with swirling to the flask containing the CuCl₂. When the CuCl₂ had dissolved, the resulting solution was diluted with 11.00 grams of deionized, distilled water and swirled.

10.00 grams of Calgon BPLF3 activated carbon (6 x 16 mesh, Calgon Carbon Corp.) were then added with agitation to coat the carbon evenly with solution, and the catalyst thus prepared was dried in air at ambient temperatures for 18 hours. The catalyst, which comprised 0.13 percent by weight of palladium on an elemental basis and 0.45 percent by weight of copper on an elemental basis on the activated carbon support, was then further air-dried in an oven at 120 degrees Celsius for 2 hours and charged, dried and reduced as described previously.

Three runs were conducted sequentially with this catalyst in the apparatus and according to the general procedure set forth just prior to Example 1 above, using a single 1.2 cubic centimeter charge of catalyst in the tubular reactor. The results from these runs are shown in Table 3 below, and suggest or show that a palladium/copper catalyst is considerably less active than the previous platinum/copper catalyst (0.25 Pt/0.45 Cu//C) of equimolar proportions for the conversion of PDC to propylene.

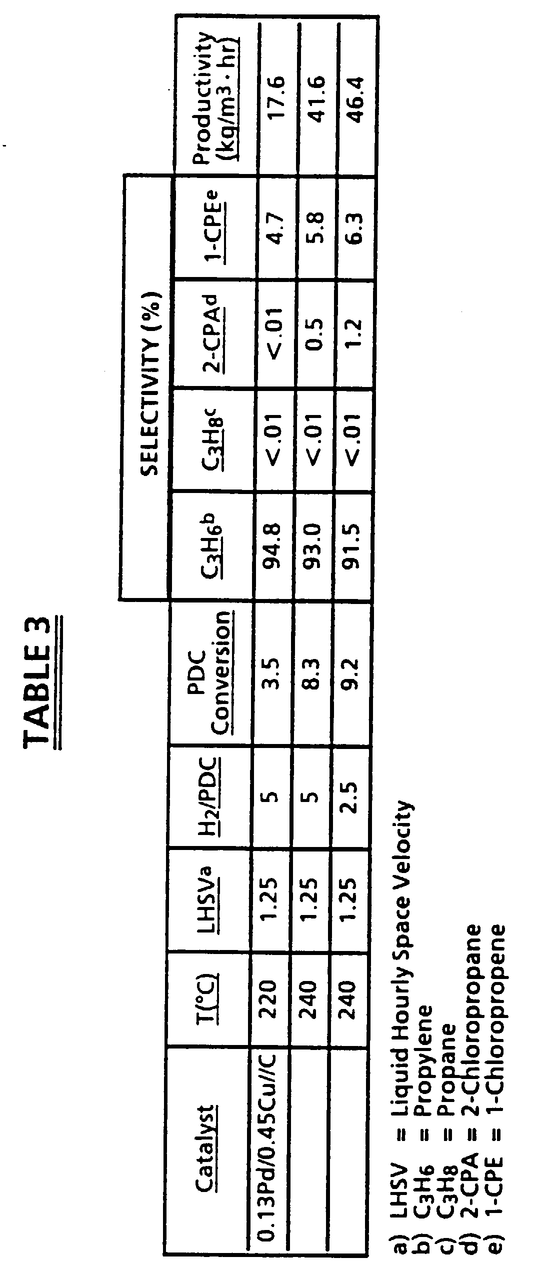

### Example 8

The catalyst for the sample runs in this example was comprised of 0.25 percent by weight of iridium (on an elemental basis) and 0.45 percent by weight of copper (again, on an elemental basis) on the same activated carbon support used in previous examples.

The iridium-copper catalyst was prepared via a stock solution of IrCl₄, by dissolving 0.911 grams of IrCl₄·xH₂O (53.4 percent iridium) in 4.988 grams of 0.1 M HCl solution in a 50.00 mL volumetric flask, and diluting this solution to 50.00 mL with deionized, distilled water.

0.095 grams of CuCl₂ (Aldrich Chemical Company, Inc., 99.999% purity) were placed in a 50 mL Erlenmeyer flask, and 2.553 grams of the IrCl₄ stock solution were added with swirling to dissolve the CuCl₂. This solution was diluted with 10.00 grams of deionized, distilled water. 10.00 grams of Calgon BPLF3 activated carbon (6 x 16 mesh; 3.4 mm x 1.2 mm) were added to the flask, and the flask agitated thoroughly to ensure that the activated carbon was evenly coated with the aqueous solution of IrCl₄ and CuCl₂. The catalyst made in this fashion, with 0.25 percent by weight of iridium (elemental basis) and 0.45 percent by weight of copper (elemental basis) on activated carbon, was air-dried as in previous examples, that is, at ambient temperature for 18 hours and then in an oven at 120 degrees Celsius for another 2 hours, and then charged to the reactor, dried and reduced.

Three runs were conducted with the catalyst in the apparatus and according to the procedures described previously, with 1.2 cubic centimeter charges of catalyst being charged to the reactor for each set of reaction conditions. The remaining reaction parameters and the results of the runs are as indicated in Table 4 below.

These results suggest or show that the iridium/copper catalyst is generally less selective for converting PDC to propylene than a like-proportioned platinum/copper catalyst.

### Example 9

The catalyst tested in this example comprised 0.29 percent by weight on an elemental basis of platinum and 0.75 percent by weight of silver on the Calgon activated carbon, and was prepared first by making an aqueous stock solution from 0.048 grams of Pt(NH₃)₄(NO₃)₂, 0.116 grams of silver nitrate, and 12.43 grams of distilled, deionized water in a 50 mL Erlenmeyer flask. The same activated carbon as used in previous examples was then added (at 9.68 grams) to the flask containing the stock solution, and the flask agitated and the catalyst dried as in previous examples. A single 1.2 cubic centimeter charge (weighing 500.1 milligrams) of the catalyst was placed in the reactor, dried and reduced, then tests were conducted sequentially with this catalyst at several hydrogen to PDC molar feed ratios.

The results from the testing are shown in Table 5 below.

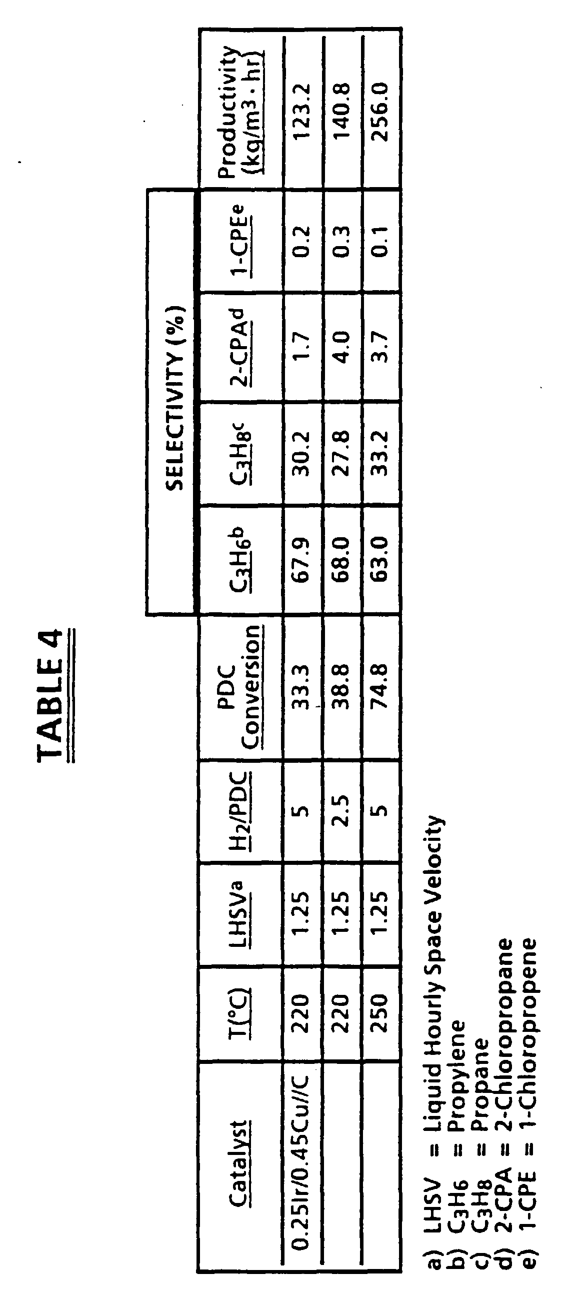

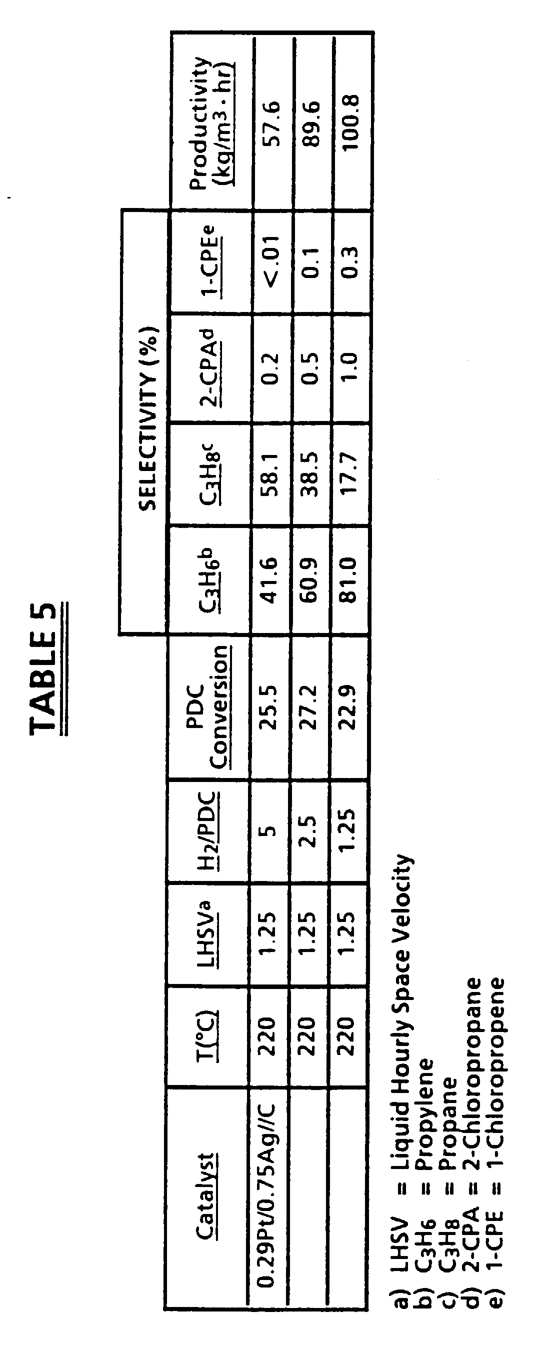

### Examples 10-13

These examples compare the effects on conversion and the selectivities to propylene and propane of pretreating a bimetallic platinum/copper catalyst by exposure to a chloride source (hydrogen chloride) without any reduction of the catalyst under hydrogen, of combining reduction under hydrogen with chloride source pretreatment, of using reduction without any chloride source pretreatment, and of simply drying the catalyst in an inert nitrogen environment and starting the process without prior exposure to a chloride source or reduction. To enable these comparisons to be made, a quantity of catalyst was initially prepared according to the general method described in Example 1 (omitting the nitrogen drying and reduction steps), but including 0.5 percent by weight of platinum and 0.9 percent by weight of copper on the Calgon BPLF3 carbon support.

A first catalyst sample (0.6 grams) taken from this quantity of catalyst was charged to the reactor by placing the catalyst on a glass wool support in the center of the reactor tubing. The catalyst was then covered with a plug of glass wool. The charged catalyst sample was thereafter dried under flowing nitrogen at 90 cubic centimeters per minute, at temperatures increasing from 25 degrees Celsius to 120 degrees Celsius at a rate of 3 degrees Celsius per minute and with the 120 degree temperature being held for an hour. The catalyst was then reduced with flowing hydrogen at 90 cubic centimeters per minute, with the temperature being raised from 120 degrees Celsius to 235 degrees Celsius at 3 degrees per minute and the 235 degree temperature being held for 2 hours. The catalyst prepared and treated in this fashion is catalyst "A" in Table 6 below.

A second sample (1.0 grams) was charged in the same manner and dried from 25 degrees Celsius to 120 degrees Celsius under flowing nitrogen, again as with the first sample. After holding at 120 degrees for one hour, however, a 50 cubic centimeter per minute flow of hydrogen chloride was introduced and maintained for one half hour. The temperature was then raised from 120 degrees to 235 degrees Celsius at 3 degrees per minute, under a reducing and chloride-source pretreating flow of 140 cc/min of a 2:1 mixture of hydrogen and hydrogen chloride. The resulting catalyst was held at 235 degrees for two hours, and is designated as catalyst "B" in Table 6 below.

A third catalyst sample (1.0 grams) was charged and dried from 25 degrees Celsius to 235 degrees Celsius at a rise of 3 degrees Celsius per minute, under flowing nitrogen at 90 cc/min. After holding for 4 hours at 235 degrees Celsius, a flow of 50 cc/min. of pure HCl was begun and maintained over an additional 4 hours. The catalyst "C" in Table 6 corresponds to this third catalyst sample.

Finally, a fourth catalyst sample (1.0 grams) was held under flowing nitrogen (at 90 cc/min.) as the temperature was raised from 25 degrees Celsius to 235 degrees Celsius at 3 degrees per minute, and then maintained at 235 degrees for 3 hours prior to startup of the process. This catalyst is catalyst "D" in Table 6.

For carrying out the conversion of PDC to propylene over these catalyst samples, PDC was pumped via a high pressure syringe pump through 1/16th inch (1.6 mm) (O.D.) Monel™ nickel alloy tubing (unless specifically noted below all of the components, tubing and fittings of the test reactor apparatus were also made of Monel™ nickel alloy (Huntington Alloys, Inco Alloys International, Inc.)) into a packed sample cylinder serving as a feed evaporator.

The 1/16th (1.6 mm) inch tubing extended almost to the center of the packed cylinder, which was heated to a vaporizing temperature of 180 degrees Celsius using electrical heat tracing. Vaporization of the feedstock was accomplished in the feed line, so that the feedstock was superheated when combined with the hydrogen feed stream. Thermocouples were used to monitor the skin temperature of the feed evaporator and the temperature of the gas exiting the feed evaporator, and the temperature of the feed evaporator was computer-controlled.

The hydrogen feed stream was metered to a preheater using a Model 8249 linear mass flow controller from Matheson Gas Products, Inc. Secaucus, N.J., with the preheater consisting of a packed sample cylinder wrapped with electrical heat tracing. Thermocouples were used to monitor both the skin temperature of the preheater and the temperature of the gas exiting the preheater. The preheater temperature was set at 140 degrees Celsius.

Vaporized feedstock exiting the evaporator was mixed with the hydrogen gas from the preheater in a 2 foot (0.61 meter) long section of 1/4 inch (0.64 cm) tubing maintained at a temperature of 140 degrees Celsius. The mixed gases then were passed into and reacted within a tubular reactor (1/2 inch (1.27 cm) O.D., 12 inches (30.5 cm) in length) located within an aluminum block heated by a cartridge heater and controlled via a computer, with about 1 hour being allowed for the reactor temperature and hydrogen gas flow to equilibrate before the flow of liquid PDC was begun.

After reacting the feedstock and hydrogen in the vapor phase in the tubular reactor thus prepared, the products from the reaction were passed to a gas sampling valve, which provided gaseous aliquots for online gas chromatographic analysis in a Hewlett-Packard Model 5890 Series II gas chromatograph (Hewlett-Packard Company). The gas chromatograph was equipped with a flame ionization detector, and used 30 meter by 0.53 millimeter (I.D.) 100 percent methyl silicone/fused silica and 30 meter by 0.53 millimeter (I.D.) porous polymer-lined fused silica columns to separate the various reaction products. Response factors were conventionally determined by injections of gravimetrically-prepared standards of the individual reaction products. These response factors were applied in conjunction with individual peak areas and the total mols of all reaction products to determine the mol percents of individual components in the reactor effluent, and the selectivity to individual reaction products.

Reaction conditions for catalysts "A" and "B" were 235 degrees Celsius, 75 psig (0.52 MPa (gauge)), a liquid hourly space velocity of 0.44 hr⁻¹, a residence time of 5 seconds and a hydrogen to PDC molar feed ratio of 3.0. Reaction conditions for catalysts "C" and "D" were somewhat different, in that the residence time was 10 seconds instead of 5 seconds and the hydrogen to PDC ratio was 1:1 instead of 3:1.

The selectivities to propylene, 2-chloropropane (2-CPa) and propane, as well as the percent of PDC converted, are as summarized in Table 6 for each of the catalysts A-D.

**Table 6**

| Catalyst | PDC Conversion (Pct.) | C₃H₈ Selectivity (Pct.) | C₃H₆ Selectivity (Pct.) | 2-CPa Selectivity (Pct.) |
|---|---|---|---|---|
| A | 68 | 48 | 40 | 12 |
| B | 67 | 2 | 86 | 12 |
| C | 26 | 0.25 | 84 | 13 |
| D | 32 | 0.42 | 86 | 11 |

### Examples 14-18

For these examples, the same apparatus and procedures were generally employed as in Examples 10-13 above to evaluate and compare several different catalyst combinations. Each of these catalysts was prepared from a solution in water of the chlorides of the metals involved, and using the Calgon BPLF3 activated carbon. The catalysts were each air- and oven--dried, charged and reduced in the manner of Examples 1-9.

The reaction temperature for this comparison was 220 degrees Celsius, the pressure was atmospheric, the residence time was 1 second, and the molar feed ratio of hydrogen to PDC was set at 3:1.

The percent conversions and the selectivities to various products (in percent) from these runs are provided in Table 7, wherein "2-CPA" is 2-chloropropane:

**Table 7**

| Catalyst | Conversion | C₃H₆ | C₃H₈ | 2-CPA |
|---|---|---|---|---|
| 0.25Pt/0.25Rh/1.3Cu | 29 | 86 | 13 | 1 |
| 0.25Pt/0.13Ru/0.9Cu | 37 | 98 | 1 | 1 |
| 0.26Ru/0.9Cu | 12 | 95 | 3 | 1 |
| 0.5Rh/1.7Cu | 19 | 95 | 3 | 1 |
| 0.5Pd/1.6Cu | 24 | 95 | 1 | 4 |

While various embodiments of the processes and catalysts of the present invention have been described and/or exemplified herein, those skilled in the art will readily appreciate that numerous changes can be made thereto which are nevertheless properly considered to be within the scope of the present invention as defined by the claims below.

## Claims

1. A process for the conversion of 1,2-dichloropropane to propylene, characterized in that 1,2-dichloropropane is reacted with hydrogen in the presence of a supported catalyst including one or more Group IB metals in elemental or compound form and one or more Group VIII metals in elemental or compound form.

2. A process as claimed in Claim 1, wherein hydrogen chloride is incorporated in the feed to the process.

3. A process as claimed in Claim 1 or Claim 2, wherein the catalyst consists of one or more Group IB metals in elemental or compound form and one or more Group VIII metals in elemental or compound form.

4. A process as claimed in Claim 3, wherein the one or more Group IB metals includes copper and wherein the one or more Group VIII metals includes platinum.

5. A process as claimed in Claim 4, wherein the Group IB and Group VIII metals consist of copper and platinum.

6. A process as claimed in Claim 5, wherein the catalyst comprises from 0.01 to 5.0 percent by weight of platinum on an elemental basis and from 0.01 to 15 percent by weight of copper, also on an elemental basis, and the catalyst support is a carbon having a specific surface area of at least 200 m²/g.

7. A process as claimed in Claim 6, wherein the catalyst comprises from 0.10 to 3.0 percent by weight of platinum on an elemental basis and from 0.05 to 5 percent by weight of copper, also on an elemental basis, and the catalyst support is a carbon having a specific surface area of at least 500 m²/g.

8. A process as claimed in Claim 7, wherein the catalyst comprises from 0.20 to 1.0 percent by weight of platinum on an elemental basis and from 0.1 to 2.0 percent by weight of copper, also on an elemental basis, and the catalyst support is a carbon having a specific surface area of at least 800 m²/g.

9. A process as claimed in any one of the preceding claims, wherein the catalyst has been pretreated by exposure to a chloride source.

10. A process as claimed in Claim 9, wherein the chloride source is hydrogen chloride.

11. A process as claimed in Claim 9 or Claim 10, wherein the catalyst is reduced prior to said pretreatment.

12. A process as claimed in any one of Claims 6 to 11, wherein the reaction is conducted in the gas phase at a pressure of from atmospheric pressure up to 10.3 MPa (gauge), at a temperature of from 100 degrees Celsius to 350 degrees Celsius, a residence time of from 0.25 seconds to 180 seconds, and a hydrogen to 1,2-dichloropropane molar feed ratio of from 0.1:1 to 100:1.

13. A process as claimed in Claim 12, wherein the reaction is conducted in the gas phase at a pressure of from 0.03 MPa (gauge) to 3.4 MPa (gauge), at a temperature of from 180 degrees Celsius to 300 degrees Celsius, a residence time of from 1.0 seconds to 20 seconds, and a hydrogen to 1,2-dichloropropane molar feed ratio of from 0.3:1 to 10:1.

14. A process as claimed in Claim 13, wherein the reaction is conducted in the gas phase at a pressure of from 0.34 MPa (gauge) to 2.1 MPa (gauge), at a temperature of from 200 degrees Celsius to 260 degrees Celsius, a residence time of from 5 seconds to 15 seconds, and a hydrogen to 1,2-dichloropropane molar feed ratio of from 0.5:1 to 3:1.

## Patentansprüche

1. Verfahren zur Umsetzung von 1,2-Dichlorpropan zu Propylen,
dadurch gekennzeichnet,
daß 1,2-Dichlorpropan in der Gegenwart eines trägergestützten Katalysators, umfassend eines oder mehrere Gruppe IB-Metalle in elementarer Form oder Verbindungsform und eines oder mehrere Gruppe VIII-Metalle in elementarer Form oder Verbindungsform mit Wasserstoff umgesetzt wird.

2. Verfahren nach Anspruch 1, wobei in der Zufuhr für das Verfahren Chlorwasserstoff aufgenommen wird.

3. Verfahren nach Anspruch 1 oder Anspruch 2, wobei der Katalysator aus einem oder mehreren Gruppe IB-Metallen in elementarer Form oder Verbindungsform und einem oder mehreren Gruppe VIII-Metallen in elementarer Form oder Verbindungsform besteht.

4. Verfahren nach Anspruch 3, wobei das oder die Gruppe IB-Metalle Kupfer umfassen und das oder die Gruppe VIII-Metalle Platin umfassen.

5. Verfahren nach Anspruch 4, wobei die Gruppe IB- und Gruppe VIII-Metalle aus Kupfer und Platin bestehen.

6. Verfahren nach Anspruch 5, wobei der Katalysator von 0,01 bis 5,0 Gewichtsprozent auf elementarer Basis Platin und von 0,01 bis 15 Gewichtsprozent, ebenfalls auf elementarer Basis Kupfer umfaßt und der Katalysatorträger ein Kohlenstoff mit einer spezifischen Oberfläche von mindestens 200 m²/g ist.

7. Verfahren nach Anspruch 6, wobei der Katalysator von 0,10 bis 3,0 Gewichtsprozent auf elementarer Basis Platin und von 0,05 bis 5 Gewichtsprozent, ebenfalls auf elementarer Basis Kupfer umfaßt und der Katalysatorträger ein Kohlenstoff mit einer spezifischen Oberfläche von mindestens 500 m²/g ist.

8. Verfahren nach Anspruch 7, wobei der Katalysator von 0,20 bis 1,0 Gewichtsprozent auf elementarer Basis Platin und von 0,1 bis 2,0 Gewichtsprozent, ebenfalls auf elementarer Basis Kupfer umfaßt und der Katalysatorträger ein Kohlenstoff mit einer spezifischen Oberfläche von mindestens 800 m²/g ist.

9. Verfahren nach einem der vorhergehenden Ansprüche, wobei der Katalysator durch Inkontaktbringen mit einer Chloridquelle vorbehandelt wurde.

10. Verfahren nach Anspruch 9, wobei die Chloridquelle Chlorwasserstoff ist.

11. Verfahren nach Anspruch 9 oder Anspruch 10, wobei der Katalysator vor der Vorbehandlung reduziert wird.

12. Verfahren nach einem der Ansprüche 6 bis 11, wobei die Reaktion in der Gasphase bei einem Druck von Atmosphärendruck bis zu 10,3 MPa (Überdruck), bei einer Temperatur von 100 Grad Celsius bis 350 Grad Celsius, einer Verweilzeit von 0,25 Sekunden bis 180 Sekunden und einem molaren Zufuhrverhältnis von Wasserstoff zu 1,2Dichlorpropan von 0,1:1 bis 100:1 durchgeführt wird.

13. Verfahren nach Anspruch 12, wobei die Reaktion in der Gasphase bei einem Druck von 0,03 MPa (Überdruck) bis 3,4 MPa (Überdruck), bei einer Temperatur von 180 Grad Celsius bis 300 Grad Celsius, einer Verweilzeit von 1,0 Sekunden bis 20 Sekunden und einem molaren Zufuhrverhältnis von Wasserstoff zu 1,2-Dichlorpropan von 0,3:1 bis 10:1 durchgeführt wird.

14. Verfahren nach Anspruch 13, wobei die Reaktion in der Gasphase bei einem Druck von 0,34 MPa (Überdruck) bis 2,1 MPa (Überdruck), einer Temperatur von 200 Grad Celsius bis 260 Grad Celsius, einer Verweilzeit von 5 Sekunden bis 15 Sekunden und einem molaren Zufuhrverhältnis von Wasserstoff zu 1,2-Dichlorpropan von 0,5:1 bis 3:1 durchgeführt wird.

## Revendications

1. Procédé pour la conversion du 1,2-dichloropropane en propylène, caractérisé en ce que l'on fait réagir le 1,2-dichloropropane avec de l'hydrogène en présence d'un catalyseur supporté comprenant un ou plusieurs métaux du Groupe IB sous forme élémentaire ou de composé et un ou plusieurs métaux du Groupe VIII sous forme élémentaire ou de composé.

2. Procédé selon, la revendication 1, dans lequel le chlorure d'hydrogène est incorporé dans l'alimentation du procédé.

3. Procédé selon la revendication 1 ou la revendication 2, dans lequel le catalyseur est constitué d'un ou plusieurs métaux du Groupe IB sous forme élémentaire ou de composé et un ou plusieurs métaux du Groupe VIII sous forme élémentaire ou de composé.

4. Procédé selon la revendication 3, dans lequel les un ou plusieurs métaux du Groupe IB comprennent le cuivre et dans lequel les un ou plusieurs métaux du Groupe VIII comprennent le platine.

5. Procédé selon la revendication 4, dans lequel les métaux du Groupe I et du Groupe VIII comprennent le cuivre et le platine.

6. Procédé selon la revendication 5, dans lequel le catalyseur se compose de 0,01 à 5,0% en poids de platine sur une base élémentaire et de 0,01 à 15% en poids de cuivre, également sur une base élémentaire, et le support de catalyseur est un carbone possédant une surface spécifique d'au moins 200m²/g.

7. Procédé selon la revendication 6, dans lequel le catalyseur se compose de 0,10 à 3,0% en poids de platine, sur une base élémentaire, et de 0,05 à 5% en poids de cuivre, également sur une base élémentaire, et le support de catalyseur est un carbone possédant une surface spécifique d'au moins 500 m²/g.

8. Procédé selon la revendication 7, dans lequel le catalyseur se compose de 0,20 à 1,0% en poids de platine, sur une base élémentaire, et de 0,1 à 2,0% en poids de cuivre, également sur une base élémentaire, et le support de catalyseur est un carbone possédant une surface spécifique d'au moins 800 m²/g.

9. Procédé selon l'une des revendications précédentes, dans lequel le catalyseur a été prétraité par une exposition à une source de chlorure.

10. Procédé selon la revendication 9, dans lequel la source de chlorure est le chlorure d'hydrogène.

11. Procédé selon la revendication 9 ou la revendication 10, dans lequel le catalyseur est réduit avant ledit prétraitement.

12. Procédé selon l'une des revendications 6 à 11, dans lequel la réaction est effectuée en phase gazeuse sous une pression comprise entre la pression atmosphérique et 10,3 MPa manométrique, à une température comprise entre 100°C et 350°C, avec une durée de séjour comprise entre 0,25 seconde et 180 secondes, et un rapport molaire d'alimentation hydrogène/1,2-dichloropropane compris entre 0,1:1 et 100:1.

13. Procédé selon la revendication 12, dans lequel la réaction est effectuée en phase gazeuse sous une pression comprise entre 0,03 MPa manométrique et 3,4 MPa manométrique, à une température comprise entre 180°C et 300°C, avec une durée de séjour comprise entre 1,0 seconde et 20 secondes, et un rapport molaire d'alimentation hydrogène/l,2-dichloropropane compris entre 0,3:1 et 10:1.

14. Procédé selon la revendication 13, dans lequel la réaction est effectuée en phase gazeuse sous une pression comprise entre 0,34 MPa manométrique et 2,1 MPa manométrique, à une température comprise entre 200°C et 260°C, avec une durée de séjour comprise entre 5 secondes et 15 secondes, et un rapport molaire d'alimentation hydrogène/1,2-dichloropropane compris entre 0,5:1 et 3:1.
